# FASCICULE DE BREVET EUROPEEN

(11) **EP 3 137 277 B1**
(45) Date de publication et mention de la délivrance du brevet: **06.06.2018**
(21) Numéro de dépôt: 15741932.6
(22) Date de dépôt: 07.04.2015
(51) Int. Cl.: B29C 49/46, B29C 49/80, A61L 2/20, B29C 49/06

(54) **MACHINE DE STÉRILISATION DE PRÉFORMES COMPORTANT UN DISPOSITIF DE DÉTECTION DE L'ENCRASSEMENT DES BUSES D'INJECTION DE FLUIDE STÉRILISANT**
MASCHINE ZUM STERILISIEREN VON VORFORMEN MIT EINER VORRICHTUNG ZUR DETEKTION VON BEWUCHS AUF DEN STERILISATIONSFLÜSSIGKEITSEINSPRITZDÜSEN
PREFORMS STERILIZING MACHINE COMPRISING A DEVICE FOR DETECTING THE FOULING OF THE STERILIZING-FLUID INJECTION NOZZLES

(30) Priorité: 30.04.2014 FR 1453931
(43) Date de publication de la demande: 08.03.2017
(73) Titulaire: Sidel Participations, 76930 Octeville-sur-Mer (FR)
(72) Inventeur: LETELLIER, Sandy, F-76930 Octeville sur Mer (FR); GIRARD, Yann, F-76930 Octeville sur Mer (FR)
(74) Mandataire: Grassin d'Alphonse, Emmanuel Jean Marie
(86) Numéro de dépôt international: PCT/EP2015/057501
(87) Numéro de publication internationale: WO 2015/165696

(56) Documents cités:
- EP-A2- 1 840 034
- DE-A1-102007 017 938
- DE-A1-102011 107 772
- US-B2- 7 806 680

## Description

La présente invention concerne une machine de stérilisation de préformes comportant un dispositif de détection de l'encrassement des buses d'injection de fluide stérilisant.

La présente invention concerne plus particulièrement une machine de stérilisation de préformes en matière plastique pour la fabrication de récipients, ladite machine comportant au moins des moyens d'alimentation en fluide stérilisant qui sont reliés sélectivement à au moins une entrée d'une conduite d'amenée dudit fluide stérilisant qui s'étend en tout ou en partie à travers une buse d'injection comportant une sortie délivrant ledit fluide stérilisant introduit à l'intérieur de ladite préforme.

On connaît du document EP-1.840.034, un exemple de machine de stérilisation de préformes en matière plastique pour la fabrication de récipients.

Une telle machine de stérilisation est notamment destinée à la mise en oeuvre du procédé de stérilisation de préformes décrit dans le document WO-2006/136498. Selon ce procédé, la stérilisation d'une préforme s'effectue par projection d'un fluide stérilisant déposé par condensation sur la paroi interne de la préforme afin de former un film de buée sensiblement uniforme propre à décontaminer ladite paroi interne.

La machine de stérilisation de préformes selon le document EP-1.840.034 est par exemple intégrée à une installation de fabrication de récipients en matière plastique, notamment de bouteilles en PET (Polyéthylène téréphtalate) destinées à recevoir des liquides alimentaires.

Dans une telle installation, la machine de stérilisation traite des préformes qui sont ensuite successivement conditionnées thermiquement dans un four, puis transformées en récipients dans des moules d'une unité de soufflage, notamment par soufflage ou par étirage-soufflage, avant d'être transférées vers une unité de remplissage et de bouchage.

Lorsque le fluide stérilisant utilisé pour stériliser est du peroxyde d'hydrogène (H₂O₂), le film de buée déposé par condensation à l'intérieur des préformes est activé thermiquement et évaporé dans le four de conditionnement thermique.

Dans une telle installation de production de récipients, on recherche par tous moyens à réduire les risques de contamination des récipients par des agents pathogènes, tels que les germes, spores, bactéries, etc. qui sont susceptibles d'affecter le contenu des récipients en les rendant notamment impropres à la consommation.

Par conséquent, il est connu d'y mettre en oeuvre différentes actions aux seules fins de contrôler et de maîtriser la qualité microbiologique de l'environnement de production.

La stérilisation des préformes constituent l'une des principales actions et la fiabilité de la stérilisation mise en oeuvre avec une machine du type précitée doit pouvoir être garantie et en conséquence contrôlée.

La demanderesse a pu établir que l'une des causes des problèmes de stérilisation de préformes rencontrés avec de telles machines de stérilisation tient parfois à l'introduction d'une quantité insuffisante de fluide stérilisant à l'intérieur des préformes.

De tels problèmes de stérilisation résultent généralement d'un encrassement de la conduite d'amenée, le plus souvent constaté en aval au niveau de la buse d'injection qui présente une section plus réduite.

La circulation du fluide stérilisant, tel que du peroxyde d'hydrogène (H₂O₂), dans la conduite d'amenée provoque la formation d'un dépôt de résidus qui engendre des perturbations de l'écoulement, voir peut l'empêcher si la conduite se retrouve quasiment obstruée.

Une solution pour garantir la stérilité du récipient serait de contrôler chaque préforme pour s'assurer de la présence du film de buée (ou condensat) de fluide stérilisant à l'intérieur de la préforme.

Si de tels contrôles sont susceptibles d'être réalisés à l'aide d'un système optique à travers la paroi de la préforme, lesdits systèmes optiques nécessaires s'avèrent complexes et surtout coûteux.

De plus, une telle méthode de contrôle ne peut être mise en oeuvre avec toutes les préformes de sorte que le problème n'est pas résolu pour certaines applications.

En effet, les systèmes optiques ne sont pas en mesure de contrôler la présence du condensat à travers la paroi de certaines préformes qui sont par exemple réalisées dans des matières plastiques opaques ou présentent une ou des couches, telle qu'au moins une couche formant une barrière contre la lumière destinée à protéger ultérieurement le produit conditionné dans le récipient.

Le but de l'invention est notamment de proposer une solution permettant de contrôler de manière simple, rapide et fiable la stérilisation des préformes effectuée au moyen d'une telle machine de stérilisation et cela quel que soit le type de préformes.

Dans ce but, l'invention propose une machine de stérilisation de préformes en matière plastique pour la fabrication de récipients du type décrit précédemment, caractérisée en ce que la machine comporte un dispositif de détection de l'encrassement d'au moins ladite une conduite d'amenée de fluide stérilisant, ledit dispositif de détection comportant au moins :
- des moyens émetteurs comportant au moins une source lumineuse émettant au moins un rayonnement lumineux qui est introduit à l'une des extrémités de la conduite d'amenée,
- des moyens récepteurs recevant ledit au moins un rayonnement lumineux à l'autre des extrémités de ladite conduite d'amenée, et
- une unité de contrôle pour déterminer le degré d'encrassement de la conduite d'amenée par comparaison entre l'intensité du rayonnement lumineux émis qui est introduit par une extrémité à l'intérieur de ladite conduite d'amenée et l'intensité du rayonnement lumineux qui est reçu par les moyens récepteurs à l'autre extrémité de la conduite d'amenée.

Avantageusement, le dispositif de détection selon l'invention est apte à contrôler l'encrassement d'une conduite d'amenée du fluide stérilisant et plus particulièrement d'une buse d'injection.

Avantageusement, au moins une étape de détection est réalisée avant d'utiliser de la machine de stérilisation en mode de fonctionnement, dit de stérilisation, dans lequel ladite machine stérilise un flux de préformes.

De préférence, la détection est réalisée de manière continue lorsque la machine de stérilisation est en mode de fonctionnement, dit de stérilisation, afin de contrôler en temps réel l'encrassement des conduites d'amenée s'étendant à travers les buses grâce à quoi le dispositif de détection permet de prévenir tout défaut de stérilisation d'une ou plusieurs préformes.

Grâce à l'invention, le contrôle de la fiabilité de la stérilisation est réalisé directement sur la machine et non pas sur les préformes stérilisées dont dès lors on s'affranchit totalement.

Selon d'autres caractéristiques de l'invention :
- ladite au moins une source lumineuse comporte au moins un composant semi-conducteur, notamment de type LED ;
- ladite au moins une source lumineuse comporte au moins un laser ;
- ledit rayonnement émis par la source présente une longueur d'onde comprise dans le domaine des infrarouges ;
- la longueur d'onde du rayonnement émis par la source est égale à 650 nm ;
- les moyens émetteurs comportent des moyens d'amplification pour amplifier sélectivement ledit rayonnement lumineux émis par ladite au moins une source ;
- le dispositif de détection comporte des moyens optiques pour faire converger ledit rayonnement lumineux émis à l'intérieur de la conduite d'amenée ;
- les moyens optiques sont constitués par au moins une lentille, notamment une lentille de Fresnel ;
- le dispositif de détection comporte des moyens conducteurs à fibre optique qui sont interposés entre les moyens émetteurs et lesdits moyens optiques ;
- le dispositif de détection comporte des moyens de comptage pour déterminer la position relative d'une buse d'injection donnée par rapport à d'autres buses d'injection de la machine.

L'invention propose également un procédé de détection de l'encrassement d'au moins une conduite d'amenée de fluide stérilisant du type qui s'étend en tout ou en partie à travers une buse d'injection équipant une machine de stérilisation de préformes en matière plastique pour la fabrication de récipients, caractérisé en ce que ledit procédé comporte au moins les étapes consistant à :
- introduire au moins un rayonnement lumineux à l'une des extrémités de ladite au moins une conduite d'amenée de fluide stérilisant,
- mesurer ledit au moins un rayonnement lumineux à l'autre des extrémités de la conduite d'amenée de fluide stérilisant,
- comparer la variation de l'intensité dudit au moins un rayonnement lumineux entre lesdites extrémités de la conduite d'amenée de fluide stérilisant afin d'en déterminer le degré d'encrassement.

D'autres caractéristiques et avantages de l'invention apparaîtront à la lecture de la description qui va suivre pour la compréhension de laquelle on se reportera aux dessins annexés dans lesquels :
- la figure 1 est une vue en perspective qui représente un exemple de réalisation d'une machine de stérilisation de préformes en matière plastique et qui illustre un mode de réalisation d'un dispositif de détection pour une telle machine ;
- la figure 2 est une vue en coupe qui représente une partie de machine et du dispositif de détection selon la figure 1 et qui illustre tout particulièrement le parcours du rayonnement lumineux à travers une conduite d'amenée du fluide stérilisant, des moyens émetteurs jusqu'aux moyens récepteurs.

Dans la description, on utilisera à titre non limitatif les expressions telles que "amont" et "aval", "supérieur" et "inférieur", "intérieur" et "extérieur", et les directions verticale et radiale en référence au repère (X, Y) représenté sur les figures et aux définitions données dans la description.

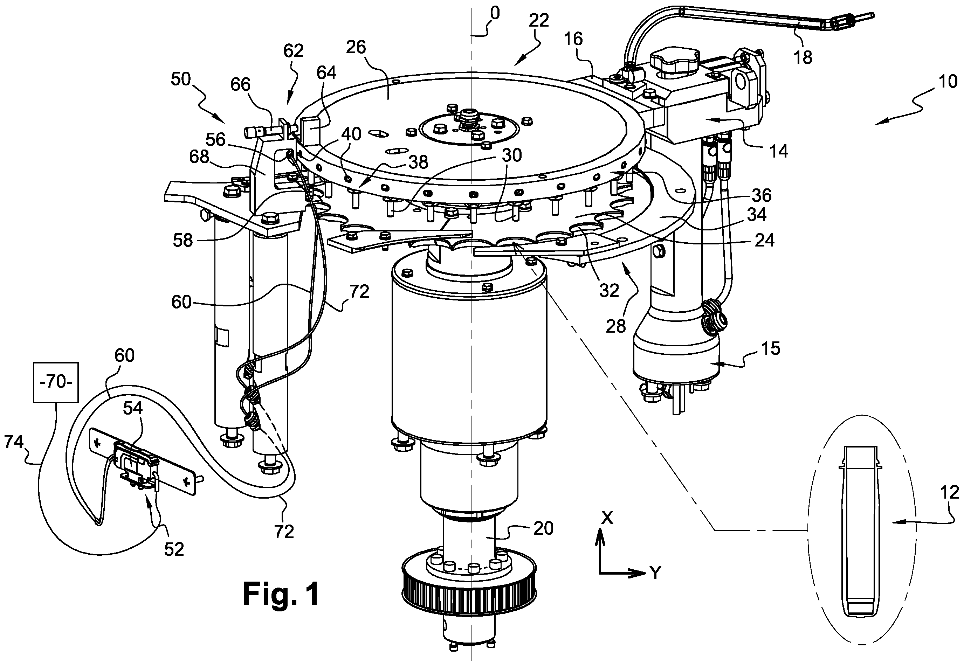

On a représenté à la figure 1, un exemple de réalisation d'une machine 10 de stérilisation de préformes 12 en matière plastique pour la fabrication de récipients, notamment de bouteilles.

Dans la suite de la présente description et par convention, l'axe X correspond à une direction verticale, déterminée sans référence aucune à la gravité terrestre, et l'axe Y correspond à une direction radiale, l'axe Y étant orthogonal à l'axe X.

De préférence, une telle préforme 12 est obtenue par moulage par injection de la matière plastique, par exemple à partir de PET.

Tel qu'illustré sur la figure 1, la préforme 12 présente un col à la forme définitive de celui du récipient, un corps destiné après conditionnement thermique à être mis en forme dans un moule, notamment par soufflage ou par étirage-soufflage.

La mise en volume de la préforme par soufflage, avec ou sans étirage, est réalisée au moyen d'au moins un fluide sous pression qui est généralement constitué par de l'air.

Avantageusement, la préforme 12 comporte à la jonction du col et du corps, une collerette qui s'étend radialement vers l'extérieur et qui est notamment utilisée lors du transport de la préforme.

La machine 10 de stérilisation selon l'exemple de réalisation est d'une conception générale analogue à celle décrite dans le document EP-1.840.034 précité auquel on pourra se reporter pour de plus amples détails.

La machine 10 de stérilisation comporte principalement une partie centrale qui, formant un convoyeur, est montée mobile en rotation autour d'un axe O de rotation, ici d'orientation verticale.

La machine 10 de stérilisation est apte à stériliser un flux de préformes 12 circulant de l'amont vers l'aval, les préformes 12 étant par exemple délivrées en entrée à la machine 10 par un dispositif d'alimentation (non représenté), stérilisées par ladite machine 10 et ensuite prises en charge en sortie par un dispositif de transfert (non représenté), tel qu'une roue munie de pinces, et cela afin d'être acheminées vers le four de conditionnement thermique d'une installation de fabrication de récipients.

Avantageusement, le fluide stérilisant utilisé par la machine 10 de stérilisation est du peroxyde d'hydrogène (H₂O₂).

Le fluide stérilisant est un mélange gazeux d'air et de peroxyde d'hydrogène (H₂O₂) qui, à l'état de vapeur sèche, est introduit au moins à l'intérieur de chaque préforme 12.

La température du mélange gazeux formant le fluide stérilisant est par exemple de l'ordre de 130°C.

De préférence, la préforme 12 est à une température inférieure à la température de condensation du fluide stérilisant de manière que le fluide stérilisant se dépose par condensation et forme un film de buée (ou condensat) qui est ensuite activé thermiquement et évaporé dans le four de conditionnement thermique.

La machine 10 de stérilisation comporte des moyens 14 d'alimentation en fluide stérilisant constitué par un bloc et comportant au moins un distributeur 16 de fluide stérilisant et une conduite 18 d'alimentation.

La machine 10 de stérilisation comporte un châssis 15 supportant une colonne centrale dans laquelle un arbre 20 d'entraînement est monté mobile en rotation autour de l'axe O de rotation et apte à être entraîné par un moteur (non représenté).

De préférence, au moins un palier est interposé radialement entre la colonne et ledit arbre 20 d'entraînement.

L'arbre 20 d'entraînement comporte à son extrémité supérieure un carrousel 22 formant ledit convoyeur et comportant au moins un plateau 24 inférieur et un plateau 26 supérieur qui sont respectivement superposés verticalement le long de l'arbre 20.

Le plateau 24 inférieur comporte des moyens 28 de support aptes à transporter, autour de l'axe O de rotation, les préformes 12 sur un parcours circulaire déterminé au cours duquel le fluide stérilisant est introduit à l'intérieur des préformes 12 en vue de les stériliser.

Pour ce faire, le plateau 26 supérieur comporte des buses 30 d'injection qui sont réparties circonférentiellement de manière régulière autour de l'axe O de rotation.

De préférence, le plateau 26 supérieur comporte des moyens de chauffage (non représentés) pour conserver la température du fluide stérilisant le traversant, le plateau 26 supérieur étant par exemple porté à une température de l'ordre de 130°C pour un mélange gazeux comportant du peroxyde d'hydrogène (H₂O₂).

Les moyens 28 de support des préformes comportent une pluralité d'encoches 32 aptes chacune à supporter une préforme 12 par l'intermédiaire de sa collerette radiale et des moyens 34 de guidage complémentaires qui sont agencés radialement à l'extérieur et en vis-à-vis du plateau 24 inférieur.

Les moyens 34 de guidage interviennent pour maintenir les préformes 12 en position lorsque le carrousel 22 est entraîné en rotation par l'arbre 20 autour de l'axe O.

Les buses 30 d'injection s'étendent verticalement, chaque buse 30 d'injection étant disposée à l'aplomb de l'une desdites encoches 32 afin que la buse 30 introduise par le col ledit fluide stérilisant à l'intérieur de la préforme 12 se trouvant en deçà.

Le nombre de buses 30 d'injection de la machine 10 de stérilisation correspond au nombre d'encoches 32 des moyens 28 de support des préformes 12.

Le nombre d'encoches 32 détermine un pas fixe entre deux préformes 12 successives.

La tranche radialement externe du plateau 26 supérieur définit une piste 36 d'alimentation des buses 30 en fluide stérilisant, ladite piste 36 d'alimentation étant de forme circulaire.

La piste 36 d'alimentation est destinée à permettre l'alimentation par le distributeur 16 d'une partie des buses 30 d'injection en fluide stérilisant.

Les moyens 14 d'alimentation sont agencés radialement à l'extérieur du carrousel 22, le distributeur 16 positionné radialement en vis-à-vis de la piste 36 d'alimentation comporte avantageusement une garniture d'étanchéité.

Le distributeur 16 coulisse radialement, orthogonalement à l'axe O de rotation, et est sollicité en direction de la piste 36 d'alimentation par des moyens d'actionnement, tels qu'un vérin pneumatique, pour plaquer ladite garniture d'étanchéité du distributeur 16 contre la piste 36 d'alimentation.

Le plateau 26 supérieur comporte une pluralité de conduites 38 d'amenée du fluide stérilisant qui sont réparties circonférentiellement, chaque conduite 38 d'amenée s'étendant en tout ou en partie à travers une buse 30 d'injection.

Chaque conduite 38 d'amenée reliée à une buse 30 d'injection comporte une entrée 40 qui débouche dans la piste 36 d'alimentation et par laquelle le distributeur 16 alimente en fluide stérilisant au moins une buse 30 d'injection associée.

De préférence, le distributeur 16 est apte à alimenter simultanément plusieurs buses 30 d'injection en introduisant ledit fluide stérilisant dans un nombre N d'entrée 40, par exemple un nombre N égal à 5.

Le distributeur 16 comporte une face interne en arc de cercle dont le rayon de courbure correspond à celui de la piste 36 d'alimentation du plateau 26 supérieur.

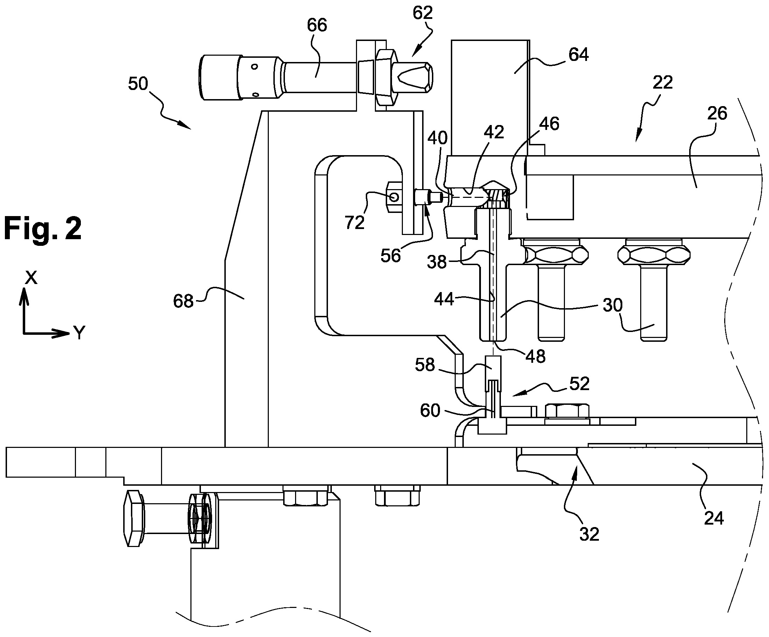

Dans l'exemple de réalisation et tel qu'illustré sur la figure 2, la conduite 38 d'amenée comporte au moins un premier tronçon 42 qui s'étend radialement et de manière rectiligne, depuis l'entrée 40 et à travers le plateau 26 supérieur puis un deuxième tronçon 44 qui s'étend verticalement et de manière rectiligne, essentiellement à travers la buse 30 d'injection.

Le premier tronçon 42 et le deuxième tronçon 44 de la conduite 38 d'amenée se raccordent par un coude 46 formant ici sensiblement un angle droit.

La buse 30 d'injection comporte à son extrémité inférieure une sortie 48 par laquelle le fluide stérilisant est introduit à l'intérieur de la préforme 12.

La machine 10 de stérilisation comporte un dispositif 50 de détection de l'encrassement d'au moins une buse 30 d'injection.

Avantageusement, le dispositif 50 de détection est apte à contrôler l'ensemble d'une conduite 38 d'amenée, depuis l'entrée 40 jusqu'à la sortie 48.

Le but du dispositif 50 de détection est de déterminer plus particulièrement si la conduite 38 d'amenée présente un encrassement tel que la stérilisation de l'intérieur des préformes 12 traitées par la buse 30 correspondante puisse s'en trouver affectée, notamment en raison de l'introduction d'une quantité insuffisante de fluide stérilisant.

Le procédé de détection de l'encrassement comporte au moins les étapes consistant à :
- introduire au moins un rayonnement lumineux à l'une 40, 48 des extrémités de ladite au moins une conduite 38 d'amenée de fluide stérilisant,
- mesurer ledit au moins un rayonnement lumineux à l'autre 40, 48 des extrémités de la conduite 38 d'amenée de fluide stérilisant,
- comparer la variation de l'intensité dudit au moins un rayonnement lumineux entre lesdites extrémités 40, 48 de la conduite 38 d'amenée de fluide stérilisant afin d'en déterminer le degré d'encrassement.

On décrira ci-après un mode de réalisation d'un dispositif 50 de détection pour la mise en oeuvre d'un tel procédé.

Le dispositif 50 de détection comporte au moins :
- des moyens 52 émetteurs comportant au moins une source 54 lumineuse émettant au moins un rayonnement lumineux qui est introduit à l'une 40, 48 des extrémités de la conduite 38 d'amenée,
- des moyens 56 récepteurs recevant ledit au moins un rayonnement lumineux à l'autre 40, 48 des extrémités de ladite conduite 38 d'amenée, et
- une unité 70 de contrôle pour déterminer le degré d'encrassement de la conduite 38 d'amenée par comparaison entre l'intensité du rayonnement lumineux émis qui est introduit par une extrémité 40, 48 à l'intérieur de ladite conduite 38 d'amenée et l'intensité du rayonnement lumineux qui est reçu par les moyens 56 récepteurs à l'autre extrémité 40, 48 de la conduite 38 d'amenée.

Dans le mode de réalisation, le dispositif 50 de détection est agencé diamétralement à l'opposé des moyens 28 d'alimentation en fluide stérilisant.

Avantageusement, le dispositif 50 d'alimentation est agencé dans une zone de la machine 10 dans laquelle les moyens 28 de support ne transportent pas de préformes 12, l'introduction desdites préformes 12 dans la machine 10 s'effectuant en aval de cette zone d'implantation du dispositif 50 d'alimentation tandis que leur évacuation après stérilisation s'effectue en amont de ladite zone.

Ainsi qu'on l'aura compris, une préforme 12 prise en charge par les moyens 28 de support de la machine 10 n'effectue pas un tour complet autour de l'axe O de rotation.

L'absence de préformes 12 facilite le contrôle par le dispositif 50 de détection de l'encrassement des différentes conduites 38 d'amenée que comporte le plateau 26 supérieur.

Avantageusement, le dispositif 50 de détection occupe une position fixe par rapport au carrousel 22 et notamment au plateau 26 supérieur qui est entraîné en rotation autour de l'axe O.

De préférence, le rayonnement lumineux est introduit par les moyens 52 émetteurs dans la conduite 38 d'amenée par la sortie 48 que comporte la buse 30 d'injection et reçu par les moyens 56 récepteurs disposés en vis-à-vis de l'entrée 40.

En variante, l'agencement des moyens 52 émetteurs et des moyens 56 récepteurs est inversé de manière que le parcours du rayonnement lumineux s'effectue de l'entrée 40 vers la sortie 48 de la conduite 38 d'amenée du fluide stérilisant.

Dans ce mode de réalisation, ladite au moins une source 54 lumineuse comporte au moins un composant semi-conducteur, notamment un composant semi-conducteur de type LED.

En variante, ladite au moins une source 54 lumineuse comporte au moins un laser.

De préférence, ledit rayonnement émis par la source 54 présente une longueur d'onde comprise dans le domaine des infrarouges, telle qu'une longueur d'onde égale à 650 nm.

Les moyens 52 émetteurs comportent des moyens d'amplification pour amplifier sélectivement ledit rayonnement lumineux émis par ladite au moins une source 54.

Avantageusement, les moyens d'amplification et la source 54 sont intégrés ensemble, les moyens d'amplification permettant de faire varier la puissance du rayonnement lumineux émis par la source 54.

Le dispositif 50 de détection comporte des moyens 58 optiques pour faire converger ledit rayonnement lumineux émis par la source 54 à l'intérieur de la conduite 38 d'amenée.

De préférence, les moyens 58 optiques sont constitués par au moins une lentille, notamment une lentille de Fresnel.

Le dispositif 50 de détection comporte des moyens 60 conducteurs qui sont interposés entre les moyens 52 émetteurs et lesdits moyens 58 optiques.

Avantageusement, les moyens 60 conducteurs sont constitués par de la fibre optique qui est apte à transmettre par réflexion totale ledit rayonnement lumineux émis par les moyens 52 émetteurs.

Grâce aux moyens conducteurs 60, les moyens 52 émetteurs et en particulier à ladite au moins une source 54 sont susceptibles d'être éloignés pour les protéger en évitant ou à tout le moins en limitant les contacts avec ledit fluide de stérilisation.

Le dispositif 50 de détection comporte des moyens 62 de comptage pour déterminer la position relative d'une buse 30 d'injection donnée par rapport à d'autres buses 30 d'injection de la machine 10 de stérilisation.

De préférence, les moyens 62 de comptage comportent au moins une cible 64 qui est solidaire en rotation du plateau 26 supérieur et un capteur 66 associé.

Le dispositif 50 de détection comporte un support 68 sur lequel est fixé ledit capteur 66 des moyens 62 de comptage.

Le support 68 est adjacent au plateau 26 supérieur et le capteur 66 disposé pour détecter face à lui chaque passage de la cible 64 lorsque le carrousel 22 est entraîné en rotation.

Avantageusement, les moyens 62 de comptage permettent de connaître la position de chacune des buses 30 d'injection afin d'identifier la ou les buses 30 dont le dispositif 50 de détection aura déterminé qu'elle(s) présente(nt) un encrassement susceptible d'affecter le résultat de l'opération de stérilisation.

En fonctionnement de la machine 10 de stérilisation, lorsque le dispositif 50 de détection identifie qu'une conduite 38 d'amenée d'une buse 30 d'injection donnée présente un encrassement de nature à affecter le résultat de l'opération de stérilisation, des moyens d'éjection (non représentés) sont alors mis en oeuvre pour éliminer sélectivement la ou les préformes ayant été traitée(s) par ladite buse 30 d'injection dont la conduite 38 d'amenée est encrassée.

De préférence, les moyens d'éjection sont disposés en aval de la machine 10 de stérilisation pour intervenir sur la ou les préformes avant leur transformation en récipients.

En variante, les moyens d'éjection sont intégrés directement à la machine 10 de stérilisation.

Avantageusement, on réalise l'élimination de toute préforme dont la stérilisation est susceptible d'être perfectible en raison d'un encrassement de la conduite 38 d'amenée de la buse 30 d'injection détecté grâce au dispositif 50 de détection.

De préférence, on procède à l'élimination de la préforme 12 après avoir effectué avec ledit dispositif 50 de détection au moins deux détections d'encrassement de la conduite 38 d'amenée incriminée.

Avantageusement, on confirme ainsi par au moins une deuxième détection tout encrassement d'une conduite 38 d'amenée identifié à l'issue d'une première détection.

En variante, l'élimination est réalisée après une seule détection, sans deuxième détection de confirmation.

Avantageusement, la machine 10 de stérilisation comporte des moyens d'alarme, par exemple sonores et/ou visuels, qui sont notamment déclenchés pour qu'une intervention soit effectuée sur la buse 30 d'injection dont la conduite 38 d'amenée aura été détectée comme présentant un degré d'encrassement déterminé.

Tel que représenté sur la figure 2, les moyens 56 récepteurs sont portés par le support 68 du dispositif 50 de détection et sont agencés face à la piste 36 d'alimentation dans laquelle découche radialement les entrées 40 des conduites 38 d'amenée du fluide stérilisant.

Les moyens 56 récepteurs sont reliés à une unité 70 de contrôle qui détermine la présence ou non d'encrassement dans une conduite 38 d'amenée en procédant, selon le procédé, à une comparaison entre l'intensité du rayonnement lumineux qui est reçu par les moyens 56 récepteurs et l'intensité du rayonnement lumineux qui est émis par les moyens 52 émetteurs.

Pour ce faire, les moyens 56 récepteurs sont reliés à l'unité 70 de contrôle par des moyens 72 de liaison, par exemple à fibre optique, afin de lui transmettre l'intensité du rayonnement lumineux mesurée par les moyens 56 récepteurs.

L'unité 70 de contrôle est également reliée aux moyens 52 émetteurs par des moyens 74 de liaison afin de connaître l'intensité du rayonnement lumineux émis par la source 54 et introduit dans la conduite 38 d'amenée par les moyens conducteurs 60 associés aux moyens 58 optiques.

Bien entendu, le dispositif 50 de détection décrit ne constitue qu'un exemple donné à titre d'exemple non limitatif.

En variante non représentée, la conduite 38 d'amenée comporte un unique tronçon rectiligne qui s'étend verticalement à travers le plateau 26 supérieur, l'entrée 40 débouche alors dans la face horizontale supérieure du plateau 26 et au moins le distributeur 16 des moyens 28 d'alimentation est modifié en conséquence alimenter les buses 30 d'injection en fluide stérilisant.

Dans une telle variante, ladite au moins une source 54 lumineuse des moyens 52 émetteurs du dispositif 50 de détection est de préférence constituée par au moins un laser.

Par comparaison avec le mode de réalisation décrit précédemment, les moyens 58 optiques sont avantageusement supprimés lorsque ladite source 54 est de type laser.

Avantageusement, la machine 10 est associée à des moyens de transfert, tels qu'une roue, qui comportent des moyens d'élimination de préformes qui sont aptes à éliminer une ou plusieurs préformes formant un lot lorsqu'un encrassement susceptible de compromettre la stérilisation de préformes a été détecté par le dispositif 50 de détection équipant ladite machine 10.

## Revendications

1. Machine (10) de stérilisation de préformes (12) en matière plastique pour la fabrication de récipients, ladite machine (10) comportant au moins des moyens (14) d'alimentation en fluide stérilisant qui sont reliés sélectivement à au moins une entrée (40) d'une conduite (38) d'amenée dudit fluide stérilisant qui s'étend en tout ou en partie à travers une buse (30) d'injection comportant une sortie (48) délivrant ledit fluide stérilisant introduit à l'intérieur de ladite préforme (12),
**caractérisée en ce que** la machine (10) comporte un dispositif (50) de détection de l'encrassement d'au moins ladite une conduite (38) d'amenée de fluide stérilisant, ledit dispositif (50) de détection comportant au moins :
- des moyens (52) émetteurs comportant au moins une source (54) lumineuse émettant au moins un rayonnement lumineux qui est introduit à l'une (40, 48) des extrémités de la conduite (38) d'amenée,
- des moyens (56) récepteurs recevant ledit au moins un rayonnement lumineux à l'autre (40, 48) des extrémités de ladite conduite (38) d'amenée, et
- une unité (70) de contrôle pour déterminer le degré d'encrassement de la conduite (38) d'amenée par comparaison entre l'intensité du rayonnement lumineux émis qui est introduit par une extrémité (40, 48) à l'intérieur de ladite conduite (38) d'amenée et l'intensité du rayonnement lumineux qui est reçu par les moyens (56) récepteurs à l'autre extrémité (40, 48) de la conduite (38) d'amenée.

2. Machine selon la revendication 1, **caractérisée en ce que** ladite au moins une source (54) lumineuse comporte au moins un composant semi-conducteur, notamment de type LED.

3. Machine selon la revendication 1, **caractérisée en ce que** ladite au moins une source (54) lumineuse comporte au moins un laser.

4. Machine selon l'une quelconque des revendications 1 à 3, **caractérisée en ce que** ledit rayonnement émis par la source (54) présente une longueur d'onde comprise dans le domaine des infrarouges.

5. Machine selon l'une quelconque des revendications 1 à 4, **caractérisée en ce que** les moyens (52) émetteurs comportent des moyens d'amplification pour amplifier sélectivement ledit rayonnement lumineux émis par ladite au moins une source (54).

6. Machine selon l'une quelconque des revendications 1 à 5, **caractérisée en ce que** le dispositif (50) de détection comporte des moyens (58) optiques pour faire converger ledit rayonnement lumineux émis à l'intérieur de la conduite (38) d'amenée.

7. Machine selon la revendication 6, **caractérisée en ce que** les moyens (58) optiques sont constitués par au moins une lentille.

8. Machine selon l'une des revendications 6 ou 7, **caractérisée en ce que** le dispositif (50) de détection comporte des moyens (60) conducteurs à fibre optique qui sont interposés entre les moyens (52) émetteurs et lesdits moyens (58) optiques.

9. Machine selon l'une quelconque des revendications 1 à 8, **caractérisée en ce que** le dispositif (50) de détection comporte des moyens (62) de comptage pour déterminer la position relative d'une buse (30) d'injection donnée par rapport à d'autres buses (30) d'injection de la machine (10).

10. Procédé de détection de l'encrassement d'au moins une conduite d'amenée de fluide stérilisant s'étendant en tout ou en partie à travers une buse d'injection équipant une machine de stérilisation de préformes en matière plastique pour la fabrication de récipients, **caractérisé en ce que** ledit procédé comporte au moins les étapes consistant à :
- introduire au moins un rayonnement lumineux à l'une des extrémités de ladite au moins une conduite d'amenée de fluide stérilisant,
- mesurer ledit au moins un rayonnement lumineux à l'autre des extrémités de la conduite d'amenée de fluide stérilisant,
- comparer la variation de l'intensité dudit au moins un rayonnement lumineux entre lesdites extrémités de la conduite d'amenée de fluide stérilisant afin d'en déterminer le degré d'encrassement.

## Patentansprüche

1. Maschine (10) zum Sterilisieren von Vorformen (12) aus Kunststoff für die Herstellung von Gefäßen, wobei die Maschine (10) mindestens Mittel (14) zur Versorgung mit Sterilisationsflüssigkeit umfasst, die selektiv mit mindestens einem Einlass (40) einer Zuleitung (38) für Sterilisationsflüssigkeit verbunden sind, die sich insgesamt oder teilweise durch eine Einspritzdüse (30) erstreckt, die einen Auslass (48) umfasst, der die ins Innere der Vorform (12) eingebrachte Sterilisationsflüssigkeit abgibt, **dadurch gekennzeichnet, dass** die Maschine (10) eine Vorrichtung (50) zur Detektion von Bewuchs an der mindestens einen Zuleitung (38) für Sterilisationsflüssigkeit umfasst, wobei die Vorrichtung (50) zur Detektion mindestens Folgendes umfasst:
- Emissionsmittel (52), umfassend mindestens eine Lichtquelle (54), die mindestens einen Lichtstrahl emittiert, der in eines (40, 48) der Enden der Zuleitung (38) eingeführt wird,
- Empfangsmittel (56), die den mindestens einen Lichtstrahl am anderen (40, 48) der Enden der Zuleitung (38) empfangen, und
- eine Überwachungseinheit (70) zur Bestimmung des Bewuchsgrades der Zuleitung (38) durch Vergleich der Intensität des emittierten Lichtstrahls, der durch ein Ende (40, 48) in das Innere der Zuleitung (38) eingeführt wird, mit der Intensität des Lichtstrahls, der von den Emfangsmitteln (56) am anderen Ende (40, 48) der Zuleitung (38) empfangen wird.

2. Maschine nach Anspruch 1, **dadurch gekennzeichnet, dass** die mindestens eine Lichtquelle (54) mindestens ein Halbleiterbauelement, insbesondere vom Typ LED, umfasst.

3. Maschine nach Anspruch 1, **dadurch gekennzeichnet, dass** die mindestens eine Lichtquelle (54) mindestens einen Laser umfasst.

4. Maschine nach einem der Ansprüche 1 bis 3, **dadurch gekennzeichnet, dass** der von der Quelle emittierte Strahl (54) eine Wellenlänge aufweist, die im Infrarotbereich liegt.

5. Maschine nach einem der Ansprüche 1 bis 4, **dadurch gekennzeichnet, dass** die Emissionsmittel (52) Verstärkungsmittel umfassen, um den von der mindestens einen Quelle (54) emittierten Strahl selektiv zu verstärken.

6. Maschine nach einem der Ansprüche 1 bis 5, **dadurch gekennzeichnet, dass** die Detektionsvorrichtung (50) Optikmittel (58) umfasst, um den emittierten Lichtstrahl im Inneren der Zuleitung (38) konvergieren zu lassen.

7. Maschine nach Anspruch 6, **dadurch gekennzeichnet, dass** die Optikmittel (58) aus mindestens einer Linse bestehen.

8. Maschine nach einem der Ansprüche 6 oder 7, **dadurch gekennzeichnet, dass** die Detektionsvorrichtung (50) Lichtwellenleitermittel (60) umfasst, die zwischen den Emissionsmitteln (52) und den Optikmitteln (58) angeordnet sind.

9. Maschine nach einem der Ansprüche 1 bis 8, **dadurch gekennzeichnet, dass** das Detektionsmittel (50) Zählmittel (62) umfasst, um die relative Position einer Einspritzdüse (30) im Verhältnis zu anderen Einspritzdüsen (30) der Maschine (10) zu bestimmen.

10. Verfahren zur Detektion von Bewuchs an mindestens einer Zuleitung für Sterilisationsflüssigkeit, die sich ganz oder teilweise durch eine Einspritzdüse erstreckt, mit der eine Maschine zum Sterilisieren von Vorformen aus Kunststoff für die Herstellung von Gefäßen ausgestattet ist, **dadurch gekennzeichnet, dass** das Verfahren mindestens die Schritte umfasst, die in Folgendem bestehen:
- Einführen mindestens eines Lichtstrahls in eines der Enden der mindestens einen Zuleitung für Sterilisationsflüssigkeit,
- Messen des mindestens einen Lichtstrahls am anderen der Enden der Zuleitung für Sterilisationsflüssigkeit,
- Vergleichen der Schwankung der Intensität des mindestens einen Lichtstrahls zwischen den Enden der Zuleitung für Sterilisationsflüssigkeit, um daraus den Bewuchsgrad zu bestimmen.

## Claims

1. Machine (10) for sterilizing plastic preforms (12) for the manufacture of containers, said machine (10) having at least means (14) for supply of sterilizing fluid, which are connected selectively to at least one inlet (40) of a conduit (38) which conveys said sterilizing fluid and extends wholly or partly through an injection nozzle (30) having an outlet (48) for delivering said sterilizing fluid introduced inside said preform (12),
**characterized in that** the machine (10) has a detection device (50) for detecting the fouling of at least said one conduit (38) for conveying sterilizing fluid, said detection device (50) having at least:
- emitter means (52) having at least one light source (54) emitting at least one light radiation which is introduced at one (40, 48) of the ends of the conveying conduit (38),
- receiver means (56) receiving said at least one light radiation at the other (40, 48) of the ends of said conveying conduit (38), and
- a control unit (70) for determining the degree of fouling of the conveying conduit (38) by comparing the intensity of the emitted light radiation introduced via one end (40, 48) inside said conveying conduit (38) and the intensity of the light radiation received by the receiver means (56) at the other end (40, 48) of the conveying conduit (38).

2. Machine according to Claim 1, **characterized in that** said at least one light source (54) has at least one semiconductor component, in particular of the LED type.

3. Machine according to Claim 1, **characterized in that** said at least one light source (54) has at least one laser.

4. Machine according to any one of Claims 1 to 3, **characterized in that** said radiation emitted by the source (54) has a wavelength in the infrared range.

5. Machine according to any one of Claims 1 to 4, **characterized in that** the emitter means (52) have amplification means for selectively amplifying said light radiation emitted by said at least one source (54).

6. Machine according to any one of Claims 1 to 5, **characterized in that** the detection device (50) has optical means (58) for causing said emitted light radiation to converge inside the conveying conduit (38).

7. Machine according to Claim 6, **characterized in that** the optical means (58) are formed by at least one lens.

8. Machine according to either of Claims 6 and 7, **characterized in that** the detection device (50) has fibre-optic conducting means (60) which are interposed between the emitter means (52) and said optical means (58).

9. Machine according to any one of Claims 1 to 8, **characterized in that** the detection device (50) has counting means (62) for determining the relative position of a given injection nozzle (30) with respect to other injection nozzles (30) of the machine (10).

10. Method for detecting the fouling of at least one conduit which conveys sterilizing liquid and extends wholly or partly through an injection nozzle of a machine for sterilizing plastic preforms for the manufacture of containers, **characterized in that** said method has at least the following steps:
- introducing at least one light radiation at one of the ends of said at least one conveying conduit for sterilizing fluid,
- measuring said at least one light radiation at the other of the ends of the conveying conduit for sterilizing fluid,
- comparing the variation of the intensity of said at least one light radiation between said ends of the conveying conduit for sterilizing fluid, so as to determine the degree of fouling therefrom.
